(19)

Europäisches
Patentamt
European
Patent Office
Office européen
des brevets

(11) **EP 4 360 543 A1**

(12) **EUROPEAN PATENT APPLICATION**

(43) Date of publication:
 **01.05.2024 Bulletin 2024/18**

(21) Application number: **22204702.9**

(22) Date of filing: **31.10.2022**

(51) International Patent Classification (IPC):
 ***A61B 5/02*** *(2006.01)* ***A61B 5/021*** *(2006.01)*
 ***A61B 5/024*** *(2006.01)* ***A61B 5/349*** *(2021.01)*
 ***G16H 50/20*** *(2018.01)*

(52) Cooperative Patent Classification (CPC):
 **A61B 5/02; A61B 5/021; A61B 5/02405;**
 **A61B 5/349; G16H 50/20**

(84) Designated Contracting States:
 **AL AT BE BG CH CY CZ DE DK EE ES FI FR GB
 GR HR HU IE IS IT LI LT LU LV MC ME MK MT NL
 NO PL PT RO RS SE SI SK SM TR**
 Designated Extension States:
 **BA**
 Designated Validation States:
 **KH MA MD TN**

(71) Applicant: **Koninklijke Philips N.V.
 5656 AG Eindhoven (NL)**

(72) Inventors:
 • **MUEHLSTEFF, Jens
 Eindhoven (NL)**

 • **WIJSHOFF, Ralph Wilhelm Christianus Gemma
 Rosa
 Eindhoven (NL)**
 • **DERKX, Rene Martinus Maria
 5656AG Eindhoven (NL)**
 • **MENA BENITO, Maria Estrella
 5656AG Eindhoven (NL)**
 • **DAVIDOIU, Valentina-Geta
 Eindhoven (NL)**
 • **HILGERS, Achim Rudolf
 Eindhoven (NL)**

(74) Representative: **Philips Intellectual Property &
 Standards
 High Tech Campus 52
 5656 AG Eindhoven (NL)**

(54) **SYSTEM AND METHOD TO DETECT AND CORRECT HEMODYNAMIC INSTABILITY IN
 HEMODYNAMIC MEASUREMENTS**

(57)      Provided is a system and method to detect and
correct hemodynamic instability occurring before, during
or after a course of hemodynamic measurements in a
subject. The system receives first measurement data cor-
responding to one or more hemodynamic parameters
from a hemodynamic measurement system. One or more
hemodynamic parameters are then derived from second
measurement data. A hemodynamic instability parame-
ter is determined based on at least one of: i) computing
a difference between the first measurement data and the
second measurement data and ii) characteristics derived
from the second measurement data. Upon determining
that the hemodynamic instability parameter exceeds a
predefined limit, the first measurement data is corrected
in the hemodynamic measurement system and reported
to a user.

Fig. 1

EP 4 360 543 A1

## Description

TECHNICAL FIELD

The present disclosure generally relates to non-invasive hemodynamic monitoring. More particularly, embodiments herein describe a system and method to detect and correct hemodynamic instability occurring before, during or after a course of hemodynamic measurements in a subject, in order to accurately and reliably measure the subject's hemodynamic parameters using a hemodynamic measurement system.

BACKGROUND

A hemodynamic measurement system such as, for instance, the ShellCuff (SC) system or the Advanced Monitoring Cuff (AMC) system is a non-invasive hemodynamic monitoring technology that can measure hemodynamic (HDM) parameters such as stroke volume (SV) and cardiac output (CO). The accuracy and precision of the SV and CO measurements of the SC or AMC system is an important aspect on which the SC or AMC system is compared to erstwhile competitive non-invasive HDM solutions.

Percentage error (PE) in CO is an important differentiator between the various CO measurements. Therefore, there is a need for a solution that will lead to more accurate measurements of SV and CO by the SC or AMC system. Furthermore, the performance of competitive solutions varies over patient populations. Therefore, it is vital to have solutions that perform steadily over various patient populations or demographics. Therefore, solutions that detect and prevent inaccurate results during HDM measurements will contribute to this objective.

The current SC measurement takes about 90 seconds. Any hemodynamic instabilities occurring during this measurement time will lead to inaccuracies in the measured SV and CO. Instabilities can occur in, for example, the heart rate (HR), respiration rate/breathing rate (RR/BR), tidal volume (TV), or blood pressure (BP). Any hemodynamic instability occurring during this measurement time affects the accuracy of the resulting SV and CO measurements which consequently may result in non-adequate medical assessment, diagnosis and/or medical therapy. Therefore, it is important to handle such instabilities for intermittent SC measurements properly. Instabilities need to be considered in terms of detection of such instabilities, for the inference of the hemodynamic parameters and for the notification of clinicians, for example, if accuracy of SV and CO parameters might be questionable.

The most common complications due to hemodynamic instability of a patient during or after a surgery are myocardial injury (MINS) and acute kidney injury (AKI). There is enough evidence that demonstrates a significant association between the degree and length of intra- and post- operative hypotension and MINS and AKI. Moreover, hypotension is the most common cause of hemodynamic instability in critically ill patients. Although death from anesthesia is rare, death within 30 days after surgery remains surprisingly common. Therefore, it is important to frequently, accurately and reliably measure a patient's blood pressure during and after surgery, which is where the SC system plays an important role. Therefore, making the measurements provided by the SC system more reliable is a key objective to be achieved.

Limitations and disadvantages of conventional and traditional approaches will become apparent to one of ordinary skill in the art, through comparison of described systems with some aspects of the present disclosure, as set forth in the remainder of the present application and with reference to the drawings.

SUMMARY

As discussed above, it is currently challenging to measure a subject's hemodynamic parameters accurately and reliably, using a hemodynamic measurement system. A claimed solution rooted in computer technology overcomes problems specifically arising in the realm of computer technology when detecting and correcting hemodynamic instability in hemodynamic measurements.

In the claimed solution of the present disclosure, a system is provided which includes a memory and a processing system communicatively coupled to the memory. The processing system is configured to receive first measurement data corresponding to one or more hemodynamic parameters from a hemodynamic measurement system. The hemodynamic measurement system is a non-invasive blood pressure measurement (NIBP) measurement system. The NIBP measurement system includes an NIBP algorithm and uses a Tissue Pressure (TP) signal and/or patient demographics for measurements. The one or more hemodynamic parameters may include, but are not limited to, stroke volume (SV), cardiac output (CO), heart rate (HR), respiration rate (RR), respiration depth, tidal volume (TV) and blood pressure (BP).

The processing system is further configured to derive one or more hemodynamic parameters from second measurement data. The second measurement data may include, but is not limited to, electrocardiography (ECG) data, bio-impedance data, pulse oximetry data, photoplethysmography (PPG) data, ventilator data, electroencephalography (EEG) data, Tissue Pressure (TP) waveforms, and PPG waveforms.

According to an aspect of the present disclosure, the processing system is configured to derive at least one of heart rate from ECG data, pulse rate from TP or PPG waveforms, breathing rate or respiration rate from ECG, bio-impedance, PPG or ventilator data, a measure of tidal volume (TV) or depth of breathing from ECG, bio-impedance, PPG or ventilator data, a change in blood pressure from a change in the pulse arrival time (PAT) as can be measured between the R-peaks in the ECG signal and the cardiac pulses in PPG, bio-impedance or TP signals, and changes in the bispectral index (BIS), which is based on the power distribution of the Fourier transform of the EEG signal.

The processing system is configured to determine a hemodynamic instability parameter based on at least one of: i) computing a difference between the first measurement data and the second measurement data and ii) characteristics derived from the second measurement data. The characteristics comprise variations in the one or more hemodynamic parameters derived from the second measurement data.

The processing system is further configured to determine if the hemodynamic instability parameter exceeds a predefined limit. Upon determining that the hemodynamic instability parameter exceeds the predefined limit, the processing system is configured to correct the first measurement data in the hemodynamic measurement system.

According to an aspect of the present disclosure, the system is configured to report the hemodynamic instability to a user.

According to another aspect of the present disclosure, the system is configured to abort and restart a measurement procedure based on the determined hemodynamic instability.

A principal object of the present disclosure is the detection of hemodynamic instabilities before, during or after the course of the measurement which further allows to (1) interrupt and restart the measurement (interruption only in cases of detection before and during the course of the measurement), or (2) report the hemodynamic instability or (3) correct the measurement for the given observed instability, or (4) avoid false alarms.

Another object of the present disclosure is to make the measurements provided by the hemodynamic measurement system more reliable.

To effectively address the issue of hemodynamic instability occurring during measurement time, the solution of the present disclosure provides an improved approach where the system automatically informs the clinician of the occurrence of hemodynamic instability, so that the clinician might decide not to use SV and CO measurements of questionable accuracy. In accordance with another aspect of the present disclosure, the system automatically corrects the inferred parameters where applicable.

Further, the solution of the present disclosure overcomes the shortcomings of the current SC system design by detecting hemodynamic instabilities occurring before, during and after the course of the hemodynamic measurements. The information about hemodynamic instability is used for assessing the accuracy of the conducted measurements. If the information indicates that a hemodynamic instability occurred, the measurement is considered inaccurate and the clinicians are made aware of this to prevent them from drawing clinical conclusions from inaccurate measurements. This further prevents drawing false conclusions and triggering false alarms on the patient monitor.

Furthermore, the solution of the present disclosure provides an improved approach that can make partial corrections for the hemodynamic instabilities occurring before, during and after the course of the measurement, as such improving the accuracy and reliability of the measured HDM parameters such as SV and CO.

It should be appreciated that all combinations of the foregoing concepts and additional concepts discussed in greater detail below (provided such concepts are not mutually inconsistent) are contemplated as being part of the subject matter disclosed herein. In particular, all combinations of claimed subject matter appearing at the end of this disclosure are contemplated as being part of the subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

BRIEF DESCRIPTION OF THE DRAWINGS

The various advantages of the embodiments will become apparent to one skilled in the art by reading the following specification and appended claims, and by referencing the following drawings, in which:

FIG. 1 is a generic block diagram illustrating an implementation of systems and/or methods described herein in accordance with an exemplary embodiment of the disclosure.

FIG. 2 is a diagram illustrating a hemodynamic instability detection and correction system in accordance with an exemplary embodiment of the disclosure.

FIG. 3 is a flowchart illustrating a method to detect and correct hemodynamic instability occurring before, during or after a course of hemodynamic measurements in a subject in accordance with an exemplary embodiment of the disclosure.

FIG. 4 is a block diagram illustrating an example computer program product in accordance with an exemplary embodiment of the disclosure.

FIG. 5 is a diagram illustrating an example of the system to detect and correct hemodynamic instability occurring before, during or after a course of hemodynamic measurements in a subject in accordance with an exemplary embodiment of the disclosure.

FIG. 6 is a diagram illustrating a semiconductor apparatus in accordance with an exemplary embodiment of the disclosure.

## DETAILED DESCRIPTION

As will be described in greater detail below, in some implementations discussed herein, a system and method is provided to detect and correct hemodynamic instability occurring before, during or after a course of hemodynamic measurements in a subject.

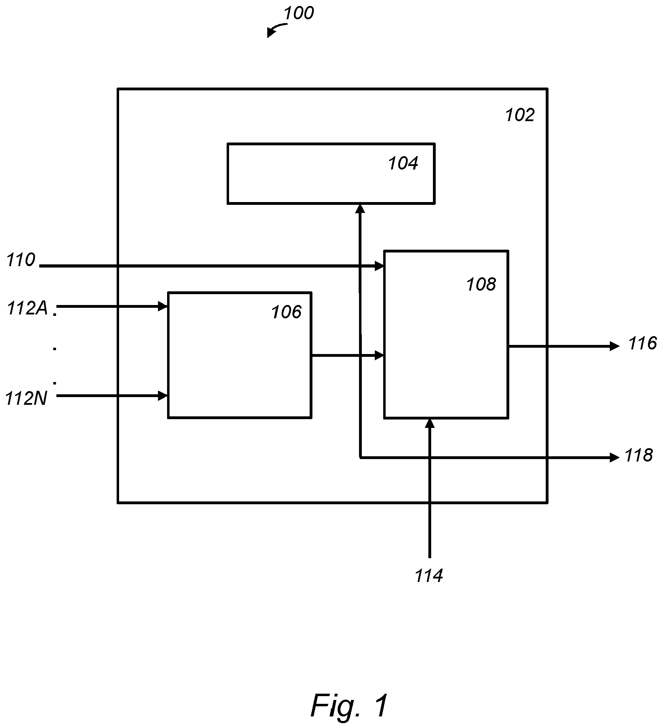

FIG. 1 is a generic block diagram illustrating an implementation of systems and/or methods described herein in accordance with an exemplary embodiment of the disclosure. Referring to FIG. 1, there is shown a system 100 that includes a hemodynamic measurement system 102, a measurement control unit 104, a hemodynamic instability detection and correction system 106, an algorithm 108, input parameters comprising Tissue Pressure (TP) signal 110, additional measurement inputs 112 (Measurements 112A-112N), and patient demographics 114, hemodynamic (HDM) parameters 116 and hemodynamic instability output 118.

The hemodynamic measurement system 102 comprises non-invasive hemodynamic monitoring technology and may further comprise suitable logic, interfaces, and/or code that may be configured to measure HDM parameters that may include, but are not limited to, stroke volume (SV) and cardiac output (CO). The hemodynamic measurement system 102 is a ShellCuff (SC) system or Advanced Monitoring Cuff (AMC) system. ShellCuff is a technology for measuring blood pressure via hard cuff. More background information of this cuff can be found in patent literature: EP2953528B1.

The hemodynamic instability detection and correction system 106 may comprise suitable logic, interfaces, and/or code that may be configured to detect and correct hemodynamic instability occurring before, during or after a course of hemodynamic measurements in a subject.

In accordance with an embodiment, the SC system uses the TP signal 110 and patient demographics 114 for its measurements. Based on additional measurement inputs 112 that may include, but are not limited to, electrocardiography (ECG) data, bio-impedance data, pulse oximetry data (photoplethysmography (PPG) data), ventilator data and electroencephalography (EEG) data (indicated by Measurement 112A ... Measurement 112N) in FIG. 1, the occurrence of hemodynamic instability may be detected shortly before, during, and shortly after the course of the measurement.

The presence of ECG, bio-impedance, PPG, ventilator and EEG data provides the following information throughout the measurement:

Heart rate (HR, from ECG) or pulse rate (PR, from the Tissue Pressure (TP) or PPG waveforms);
Breathing rate (BR) or respiration rate (RR), may be obtained from ECG, bio-impedance, PPG or ventilator data;
A measure of tidal volume (TV) or depth of breathing may be obtained from ECG, bio-impedance, PPG or ventilator data;
A change in blood pressure can be determined from, for example, a change in the pulse arrival time (PAT) as can be measured between the R-peaks in the ECG signal and the cardiac pulses in PPG, bio-impedance or TP signals.
Changes in the bispectral index (BIS), which is based on the power distribution of the Fourier transform of the EEG signals, that has been shown to be correlated with changes in hemodynamic parameters.

In accordance with an embodiment, any significant variation in HR (or PR), RR/BR, the measure of TV, BP or EEG-derived parameters such as BIS can be considered a sign of hemodynamic instability. If hemodynamic instability is detected:

This information may be reported to a user as hemodynamic instability output 118, so the user can take this into account when assessing the results.

This information can be used in the ShellCuff algorithm 108 to correct for the effect of the hemodynamic instability and output corrected HDM parameters 116.

This information can be used by the ShellCuff measurement control unit 104 to abort and restart the measurement procedure.

In accordance with another embodiment, the additional measurements proposed above to assess hemodynamic instability can also be used with a traditional air-filled cuff for non-invasive blood pressure (NIBP) measurements. That is, before, during and after the course of a traditional NIBP measurement, the additional measurements can be used to detect the occurrence of hemodynamic instability. If hemodynamic instability is detected:
This information can be reported to the user as the hemodynamic instability output 118, so the user can take this into account when assessing the results.

This information can be used in the NIBP algorithm 108 to correct for the effect of the hemodynamic instability and output corrected HDM parameters 116.

This information can be used by the NIBP measurement control unit 104 to abort and restart the measurement procedure.

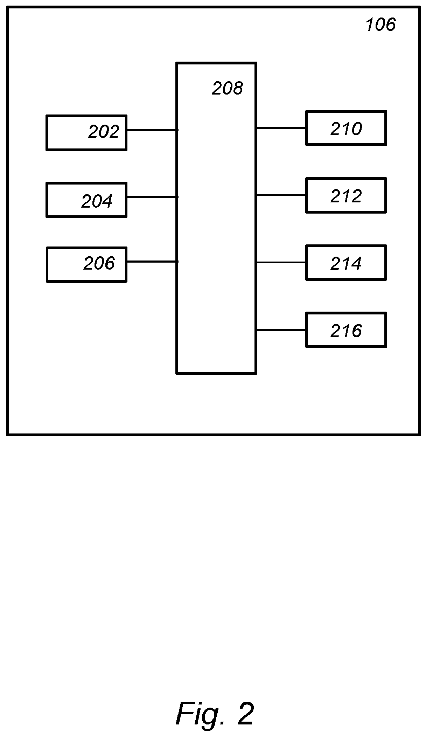

FIG. 2 is a diagram illustrating a hemodynamic instability detection and correction system in accordance with an exemplary embodiment of the disclosure. Referring to FIG. 2, there is shown the hemodynamic instability detection and correction system 106 that includes a memory 202, a processing system 204, an input component 206, a communications unit 208, a derivation component 210, a detection component 212, a correction component 214 and an output component 216.

The memory 202 may include, but is not limited to, one or more memory devices, persistent storage, computer-readable storage media, random access memory (RAM) and cache memory. In general, the memory 202 may include any suitable volatile or non-volatile computer-readable storage media. The memory 202 may comprise suitable logic, and/or interfaces, that may be configured to store instructions (for example, computer readable program code) that can implement various aspects of the present disclosure.

The memory 202 is communicatively coupled to the input component 206, the processing system 204, and the output component 216.

The processing system 204 may comprise suitable logic, interfaces, and/or code that may be configured to execute the instructions stored in the memory 202 to implement various functionalities of the hemodynamic instability detection and correction system 106 in accordance with various aspects of the present disclosure. The processing system 204 may be further configured to communicate with various modules of the hemodynamic instability detection and correction system 106 via the communications unit 208.

The communications unit 208 may be configured to transmit data between modules, engines, databases, memories, and other components of the hemodynamic instability detection and correction system 106 for use in performing the functions discussed herein. The communications unit 208 may include one or more communication types and utilize various communication methods for communication within the hemodynamic instability detection and correction system 106.

The input component 206 may comprise suitable logic, interfaces, and/or code that may be configured to receive first measurement data corresponding to one or more hemodynamic parameters from the hemodynamic measurement system 102. The hemodynamic measurement system 102 is a non-invasive blood pressure measurement (NIBP) measurement system. The NIBP measurement system includes an NIBP algorithm (the algorithm 108) and uses the Tissue Pressure (TP) signal 110 and/or patient demographics 114 for measurements. The one or more hemodynamic parameters may include, but are not limited to, stroke volume (SV), cardiac output (CO), heart rate (HR), respiration rate (RR), respiration depth, tidal volume (TV) and blood pressure (BP).

The derivation component 210 may comprise suitable logic, interfaces, and/or code that may be configured to derive one or more hemodynamic parameters from second measurement data. The second measurement data may include, but is not limited to, electrocardiography (ECG) data, bio-impedance data, pulse oximetry data, photoplethysmography (PPG) data, ventilator data, electroencephalography (EEG) data, Tissue Pressure (TP) waveforms, and PPG waveforms.

According to an aspect of the present disclosure, the derivation component 210 is configured to derive at least one of heart rate from ECG data, pulse rate from TP or PPG waveforms, breathing rate or respiration rate from ECG, bio-impedance, PPG or ventilator data, a measure of tidal volume (TV) or depth of breathing from ECG, bio-impedance, PPG or ventilator data, a change in blood pressure from a change in the pulse arrival time (PAT) as can be measured between the R-peaks in the ECG signal and the cardiac pulses in PPG, bio-impedance or TP signals, and changes in the bispectral index (BIS), which is based on the power distribution of the Fourier transform of the EEG signal.

The detection component 212 may comprise suitable logic, interfaces, and/or code that may be configured to determine a hemodynamic instability parameter based on at least one of: i) computing a difference between the first measurement data and the second measurement data and ii) characteristics derived from the second measurement data. The characteristics comprise variations in the one or more hemodynamic parameters derived from the second measurement data.

The detection component 212 is further configured to determine if the hemodynamic instability parameter exceeds a predefined limit.

The correction component 214 may comprise suitable logic, interfaces, and/or code that may be configured to correct the first measurement data in the hemodynamic measurement system based on receiving an input from the detection component 212 that the hemodynamic instability parameter exceeds the predefined limit.

The output component 216 may comprise suitable logic, interfaces, and/or code that may be configured to report the hemodynamic instability to a user.

Furthermore, the hemodynamic instability detection and correction system 106 is configured to abort and restart a measurement procedure based on the determined hemodynamic instability.

In accordance with an embodiment, the detection, assessment and reporting of the presence of hemodynamic instability during the course of the ShellCuff measurement is described.

Additional measurements (Measurement 112A .... Measurement 112N) may be used by the ShellCuff system to assess hemodynamic instability during the measurement. Additional inputs such as, but not limited to, ECG, bio-impedance and pulse oximetry data (photoplethysmography (PPG) data) may be used to determine whether the patient's heart rate, respiration rate, respiration depth and blood pressure are stable throughout the measurement. The PPG data may be obtained from various body sites, such as peripherally at the finger, foot or earlobe, or more centrally from the alar wing, nasal septum, ear concha or the forehead.

Presence of ECG, bio-impedance and PPG data provides the following information throughout the measurement:

By assessing the beat-to-beat variation in the R-peaks in the ECG signal or the cardiac pulses in the TP or PPG signals, it can be assessed whether the heart/pulse rate is stable throughout the course of the measurement or whether arrhythmias occurred.

By comparing the PR determined from the TP signal to the PR obtained from a PPG signal or the HR obtained from an ECG signal, the reliability of the TP signal can be assessed. If the difference between the PR from the TP signal and the PR from the PPG signal or the HR from the ECG signal exceeds a predefined limit, i.e., if $|PR_{TP} - PR_{PPG}| > PR_{lim}$ or $|PR_{TP} - HR_{ECG}| > PR_{lim}$, this can also be used as an indication that the SC measurement may not be reliable.

Breathing cycles may be assessed by analyzing the ECG, bio-impedance and/or PPG signals. By assessing variation in the duration of individual breathing cycles, it can be assessed whether the breathing rate is stable during the course of the measurement. By assessing the amplitude of the individual breathing cycles, a measure of the depth of the individual breathing cycles may be obtained, from which it can be determined whether the respiration depth has been stable during the course of the measurement.

By measuring pulse arrival time (PAT) between, for example, the R-peaks in the ECG signal and the individual cardiac pulses in the TP signal or the PPG signal acquired from, for example, a central location such as, but not limited to, the alar wing, nasal septum, forehead or ear concha, or from a peripheral location such as the finger, earlobe or foot, a surrogate measure for variations in blood pressure can be obtained. If the PAT remains stable throughout the course of the measurement, i.e. if the variation in PAT throughout the course of measurement remains below a predefined threshold, it can be assumed that the blood pressure has remained stable during the course of the measurement.

If an instability is detected before or during the course of the measurement in any of the HR/PR, RR, respiration depth or blood pressure/PAT parameters explained above, the instability will be reported to the user, so the user is aware of these instabilities when assessing the HDM results provided by ShellCuff

In the embodiment described above, a traditional NIBP measurement may be used instead of the Shell-Cuff system.

In accordance with another embodiment, the detection, assessment and reporting of the presence of hemodynamic instability during a ShellCuff measurement based on ventilator data, is described.

Additional related context factors are taken into account that can influence the hemodynamic stability of a patient and can consequently affect the ShellCuff measurements. For instance, if a patient is on mechanical ventilation, the ventilator can measure whether the patient follows the ventilation rate and depth imposed by the ventilator or whether the patient deviates from the imposed ventilation rate or depth. Alternatively, if a patient is spontaneously breathing and only requires support by the ventilator, the ventilator can continuously measure the spontaneous respiration rate and depth of the patient. Therefore, in this scenario (1) ventilator signals such as airway pressure and flow can be used by the ShellCuff system to detect any irregularities in the respiration rate or depth of the patient, or (2) the ventilation rate and depth parameters as measured by the ventilator can be used by the ShellCuff system to detect any irregularities in the respiration rate or depth of the patient. Also in this scenario, respiration information can be collected before, during and after the course of the SC measurements to detect any instabilities in the respiration/ventilation rate or depth.

If an instability is detected before or during the course of the measurement in the respiration rate or depth, this instability will be reported to the user, so the user is aware of these instabilities when assessing the HDM results provided by ShellCuff.

In the embodiment described above, a traditional NIBP measurement may be used instead of the Shell-Cuff system.

In accordance with yet another embodiment, the detection, assessment and reporting of the presence of hemodynamic instability during a ShellCuff measurement based on electroencephalography (EEG) measurements, is described.

The above mentioned parameters are directly correlated to the heart function and activity (ECG, bio-impedance or pulse oximetry) but also may be influenced by other body-functions such as, for example, the respiratory system (pulse oximetry) or environmental conditions such as, for example, the environmental temperature (sweating would influence bio-impedance). Accordingly, a combination of different effects can have an impact on the blood pressure and other hemodynamic parameters of the vascular system, that are directly depending on each other. However, it is of additional benefit if another physiologic parameter (and measurement) can be used, that is not directly correlated to the hemodynamics of a human being, hence a more independent parameter to assess possible hemodynamic instability during a ShellCuff measurement.

Electroencephalography (EEG) measurements can be used to identify changes of hemodynamic parameters. Amongst others, the bispectral index (BIS, which is based on the power distribution of the Fourier transform of the EEG signals), is compared to measurements of the heart rate (HR) and mean arterial blood pressure (MAP).

Therefore in another embodiment, it is proposed to use electroencephalography (EEG) measurements, to assess possible hemodynamic instabilities during a SC measurement. Required EEG electrodes could be directly connected to the patient monitor where the ShellCuff is connected, or to a separate EEG scanner which could communicate with the SC-PM (ShellCuff-Patient Monitor) or could be monitored by the operator.

If an instability is detected before or during the course of the measurement in the EEG-derived parameter, such as a BIS, this will be reported to the user, so the user is aware of these instabilities when assessing the HDM results provided by ShellCuff.

In this embodiment, a traditional NIBP measurement may be used instead of the ShellCuff system.

In accordance with yet another embodiment, an automatic action of the system is disclosed. This includes automatically stopping and restarting of the measurement in case of detection of hemodynamic instabilities.

If a hemodynamic instability is detected in any of the mentioned parameters during the course of the measurement, the system can also automatically abort and restart the measurement procedure, by providing the instability detections to the measurement control unit 104. Alternatively, the measurement control unit 104 can abort the measurement upon receiving a detection of hemodynamic instability and then request for user input to restart the measurement.

In accordance with yet another embodiment, corrections are made to detected hemodynamic instabilities. Corrections can be made to the ShellCuff measurements, upon the detection of hemodynamic instabilities. For instance, these corrections may be made during the course of the measurement or retrospectively after a full measurement cycle of the ShellCuff system has been completed. Various kinds of hemodynamic instabilities can be detected and corrected for:

In case arrhythmias are detected from the ECG, PPG or TP waveforms, such as missing beats or premature beats, these beats can be corrected in the TP waveform by interpolation of neighboring normal beats in the TP waveform to correct for the missing or premature beats. This then allows to further run the ShellCuff algorithm 108 to determine SV, CO and other HDM parameters. The correction of missing or premature beats can only be done if the number of missing or premature beats is limited over the duration of the recording as normal neighboring beats are required for the correction. In this case, a traditional NIBP measurement may be used instead of the ShellCuff system, where corrections are then made to the cuff-pressure signal.

ShellCuff determines the mean arterial pressure (MAP) as the clamping pressure in relation to the occurrence of the maximum oscillation. The systolic and diastolic pressures are also determined in relation to the occurrence of the maximum oscillation.

If the blood pressure increases during the course of the measurement, the maximum of the envelope will move to the right, as a result of which the mean, systolic and diastolic pressures are overestimated. If the blood pressure decreases during the course of the measurement, the envelope will start to decay sooner, as a result of which the mean, systolic and diastolic pressures will be underestimated. As these pressures are used in the determination of SV and CO, this will also affect the resulting SV and CO. The SV and consequently the CO estimated by the ShellCuff are inversely related to the mean arterial pressure (MAP). Therefore, if the MAP is overestimated, this will lead to an underestimation of SV and CO and if the MAP is underestimated, this will lead to an overestimation of SV and CO.

By making use of the Pulse Arrival Time (PAT) as measured, it can be determined whether the blood pressure is stable during the course of the measurement, or whether the blood pressure increases or decreases during the course of the measurement. By determining the difference in PAT over the course of the measurement as $\Delta PAT = PAT_{end} - PAT_{beginning}$, the following situations may be distinguished:

If the PAT is stable throughout the course of the measurement, i.e. $\Delta PAT = 0$ or $\Delta PAT$ is below a predefined threshold $|\Delta PAT| < \Delta PAT_{lim}$, this confirms that the blood pressure remained stable during the course of the measurement and the resulting SV and CO will be accurate and can be trusted.

If the PAT increases during the course of the measurement, i.e. $\Delta PAT > 0$ or $\Delta PAT > \Delta PAT_{lim}$, this indicates that the blood pressure decreased during the course of the measurement and the mean and systolic pressure are underestimated and the SV and CO are overestimated.

If the PAT decreases during the course of the measurement, i.e. $\Delta PAT < 0$ or $\Delta PAT < \Delta PAT_{lim}$, this indicates that the blood pressure increased during the course of the measurement and the mean and systolic pressure are overestimated and the SV and CO are underestimated.

By making use of the variation in PAT during the course of the measurement, as indicated by $\Delta PAT$, the SV and CO measurement can be corrected for the resulting variation in blood pressure, via for example:

$$SV_{corrected} = SV - \alpha_{corr}\,\Delta PAT,$$

$$CO_{corrected} = PR \times SV_{corrected},$$

in which $\alpha_{corr}$ is a positive correction factor.

In case corrections are being applied by the algorithm 108, this is also reported to the user to make the user aware of the corrections so these can be taken into account in the assessment of the results.

In this case, correcting the measured NIBP parameters may also be considered, based on the measured $\Delta PAT$. That is, correcting systolic, mean or diastolic blood pressure may be considered as:

$$BP_{corrected} = BP + \beta_{corr}\Delta PAT,$$

where BP represents any of systolic, mean or diastolic pressure and $\beta_{corr}$ is a positive correction factor. In the context of the correction of BP parameters, either the ShellCuff system or a traditional NIBP measurement system may be used.

In accordance with yet another embodiment, assessment of hemodynamic stability prior to starting a measurement to recommend the most appropriate measurement mode with the ShellCuff system, is described.

The additional measurement data such as ECG, bio-impedance, pulse oximetry / PPG, ventilator and EEG data are used to assess the patient's hemodynamic stability prior to the measurement. Upon starting a measurement with the ShellCuff system, the following actions can be taken, if the additional measurements show that the patient is hemodynamically unstable:

In one embodiment, the ShellCuff system can recommend to not initiate the full measurement, because the patient is hemodynamically unstable.

In another embodiment, the ShellCuff system has a Fast Measurement Mode in addition to the Full Measurement Mode and can recommend to switch to the Fast Measurement Mode which is appropriate to use during hemodynamic instability. E.g., the ShellCuff system can have a Fast Measurement Mode which supports only a limited number of parameters which can be measured with the required accuracy within a period of time shorter than 90 s. That is, the Fast Measurement Mode measures a reduced number of parameters compared to the Full Measurement Mode, where the Full Measurement Mode requires about 90 s of measurement time.

The approach and solutions of this embodiment can also be extended to a traditional air-filled cuff NIBP measurement, where also the possibility can be offered to measure a reduced parameter set with the required accuracy in a shortened period of time.

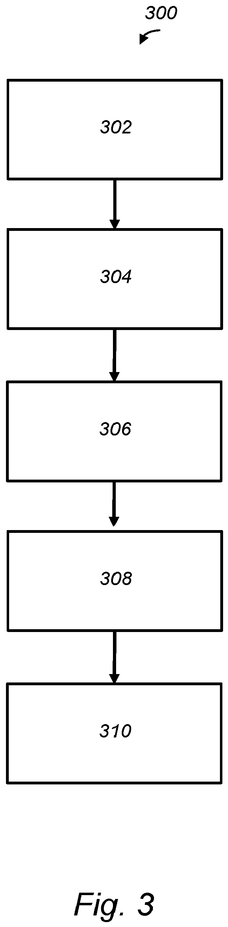

FIG. 3 is a flowchart illustrating a method to detect and correct hemodynamic instability occurring before, during or after a course of hemodynamic measurements in a subject in accordance with an exemplary embodiment of the disclosure. Referring to FIG. 3, there is shown a flowchart of a method 300 to detect and correct hemodynamic instability occurring before, during or after a course of hemodynamic measurements in a subject.

At 302, receive first measurement data corresponding to one or more hemodynamic parameters from a hemodynamic measurement system. The input component 206 is configured to receive first measurement data corresponding to one or more hemodynamic parameters from the hemodynamic measurement system 102. The one or more hemodynamic parameters may include, but are not limited to, stroke volume (SV), cardiac output (CO), heart rate (HR), respiration rate (RR), respiration depth, tidal volume (TV) and blood pressure (BP).

At 304, derive one or more hemodynamic parameters from one or more second measurement data. The derivation component 210 is configured to derive one or more hemodynamic parameters from second measurement data. The second measurement data may include, but is not limited to, electrocardiography (ECG) data, bio-impedance data, pulse oximetry data, photoplethysmography (PPG) data, ventilator data, electroencephalography (EEG) data, Tissue Pressure (TP) waveforms, and PPG waveforms.

At 306, determine a hemodynamic instability parameter based on at least one of: i) computing a difference between the first measurement data and the second measurement data and ii) characteristics derived from the second measurement data. The detection component 212 is configured to determine a hemodynamic instability parameter based on at least one of: i) computing a difference between the first measurement data and the second measurement data and ii) characteristics derived from the second measurement data. The characteristics comprise variations in the one or more hemodynamic parameters derived from the second measurement data.

At 308, determine if the hemodynamic instability parameter exceeds a predefined limit. The detection component 212 is configured to determine if the hemodynamic instability parameter exceeds a predefined limit.

At 310, upon determining that the hemodynamic instability parameter exceeds the predefined limit, correct the first measurement data in the hemodynamic measurement system. The correction component 214 is configured to correct the first measurement data in the hemodynamic measurement system based on receiving an input from the detection component 212 that the hemodynamic instability parameter exceeds the predefined limit.

Many different ways of executing the method described above are possible, as will be apparent to a person skilled in the art. For example, the order of the steps can be performed in the shown order, but the order of the steps can be varied, or some steps may be executed in parallel. Moreover, in between steps other method steps may be inserted. The inserted steps may represent refinements of the method such as described herein or may be unrelated to the method. For example, some steps may be executed, at least partially, in parallel. Moreover, a given step may not have finished completely before a next step is started.

Embodiments of the method may be executed using software, which comprises instructions for causing a processor system to perform method 300. Software may only include those steps taken by a particular subentity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

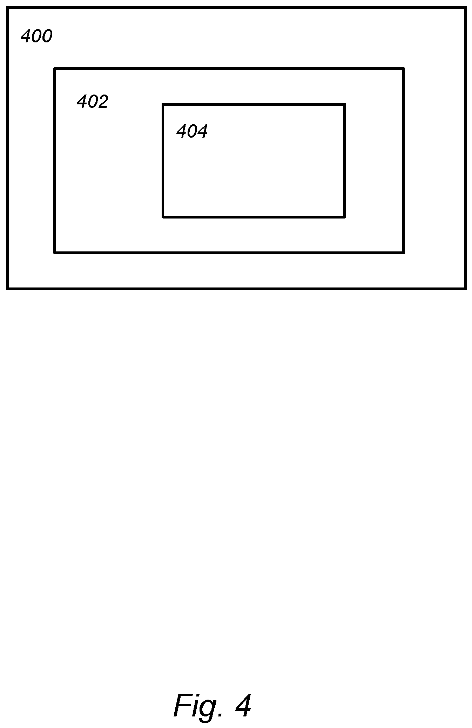

FIG. 4 is a block diagram illustrating an example computer program product in accordance with an exemplary embodiment of the disclosure. Referring to FIG. 4, there is shown a computer program product 400 that includes a machine-readable storage 402 that may also include logic 404. In some implementations, the machine-readable storage 402 may be implemented as a non-transitory machine-readable storage. In some implementations, the logic 404 may be implemented as machine-readable instructions, such as software, for example. In an embodiment, the logic 404, when executed, implements one or more aspects of the method 300 (FIG. 3), and/or realize the system 100 (FIG. 1), already discussed.

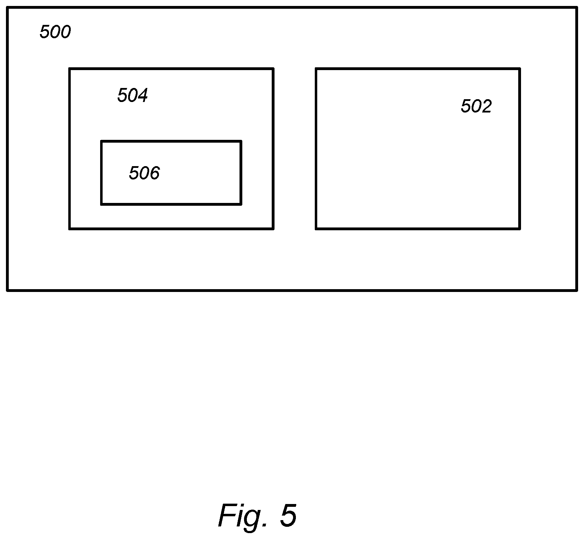

FIG. 5 is a diagram illustrating an example of the system to detect and correct hemodynamic instability occurring before, during or after a course of hemodynamic measurements in a subject in accordance with an exemplary embodiment of the disclosure. Referring to FIG. 5, in the illustrated example, the system 500 may include a processor 502 and a memory 504 communicatively coupled to the processor 502. The memory 504 may include logic 506 as a set of instructions. In some implementations, the logic 506 may be implemented as software. In an embodiment, the logic 506, when executed by the processor 502, implements one or more aspects of the method 300 (FIG. 3), and/or realize the system 100 (FIG. 1), already discussed.

In some implementations, the processor 502 may include a general-purpose controller, a special purpose controller, a storage controller, a storage manager, a memory controller, a microcontroller, a general-purpose processor, a special purpose processor, a central processor unit (CPU), the like, and/or combinations thereof.

Further, implementations may include distributed processing, component/object distributed processing, parallel processing, the like, and/or combinations thereof. For example, virtual computer system processing may implement one or more of the methods or functionalities as described herein, and the processor 502 described herein may be used to support such virtual processing.

In some examples, the memory 504 is an example of a computer-readable storage medium. For example, memory 504 may be any memory which is accessible to the processor 502, including, but not limited to RAM memory, registers, and register files, the like, and/or combinations thereof. References to "computer memory" or "memory" should be interpreted as possibly being multiple memories. The memory may for instance be multiple memories within the same computer system. The memory may also be multiple memories distributed amongst multiple computer systems or computing devices.

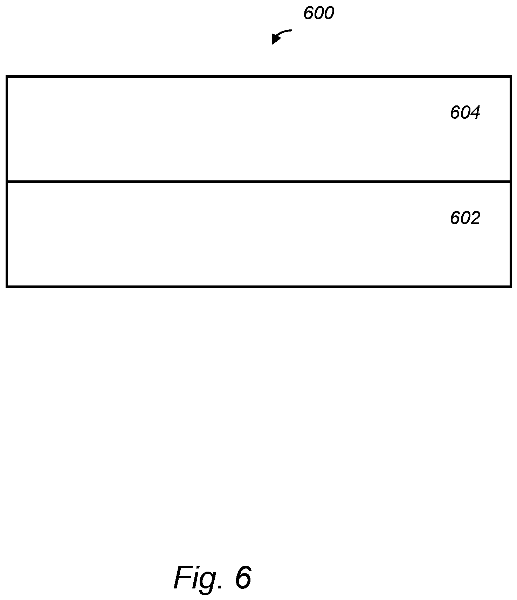

FIG. 6 is a diagram illustrating a semiconductor apparatus in accordance with an exemplary embodiment of the disclosure. The illustrated semiconductor apparatus 600 (e.g., chip and/or package) includes one or more substrates 602 (e.g., silicon, sapphire, or gallium arsenide) and logic 604 (e.g., configurable logic and/or fixed-functionality hardware logic) coupled to the substrate(s) 602. In an embodiment, the logic 604 implements one or more aspects of the method 300 (FIG. 3), and/or realize the system 100 (FIG. 1), already discussed.

In some implementations, the logic 604 may include a transistor array and/or other integrated circuit/IC components. For example, configurable logic and/or fixed-functionality hardware logic implementations of the logic 604 may include configurable logic such as, for example, programmable logic arrays (PLAs), field programmable gate arrays (FPGAs), complex programmable logic devices (CPLDs), or fixed-functionality logic hardware using circuit technology such as, for example, application specific integrated circuit (ASIC), complementary metal oxide semiconductor (CMOS) or transistor-transistor logic (TTL) technology, the like, and/or combinations thereof.

All definitions, as defined and used herein, should be understood to control over dictionary definitions, definitions in documents incorporated by reference, and/or ordinary meanings of the defined terms.

The subject matter described herein sometimes illustrates different components contained within, or connected with, different other components. It is to be understood that such depicted architectures are merely exemplary, and that in fact many other architectures can be implemented which achieve the same functionality. In a conceptual sense, any arrangement of components to achieve the same functionality is effectively "associated" such that the desired functionality is achieved. Hence, any two components herein combined to achieve a particular functionality can be seen as "associated with" each other such that the desired functionality is achieved, irrespective of architectures or intermedial components. The term "coupled" may be used herein to refer to any type of relationship, direct or indirect, between the components in question, and may apply to electrical, mechanical, fluid, optical, electromagnetic, electromechanical, or other connections. Likewise, any two components so associated can also be viewed as being "operably connected", or "operably coupled", to each other to achieve the desired functionality, and any two components capable of being so associated can also be viewed as being "operably couplable", to each other to achieve the desired functionality. Specific examples of operably couplable include but are not limited to physically mateable and/or physically interacting components.

In the claims, as well as in the specification above, the terms "first", "second", etc. may be used herein only to facilitate discussion, and carry no particular temporal or chronological significance unless otherwise indicated.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

The indefinite articles "a" and "an," as used herein in the specification and in the claims, unless clearly indicated to the contrary, should be understood to mean "at least one."

As used herein, the term "or" or "and/or" is inclusive and not exclusive, unless expressly indicated otherwise or indicated otherwise by context. Therefore, herein, "A or B" means "A, B, or both," unless expressly indicated otherwise or indicated otherwise by context. Moreover, "and" is both joint and several, unless expressly indicated otherwise or indicated otherwise by context. Therefore, herein, "A and B" means "A and B, jointly or severally," unless expressly indicated otherwise or indicated otherwise by context.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used in this application and in the claims, a list of items joined by the term "one or more of' may mean any combination of the listed terms. For example, the phrases "one or more of A, B or C" may mean A; B; C; A and B; A and C; B and C; or A, B and C.

As is described above in greater detail, one or more processor, other unit, the like, and/or combinations thereof may fulfill the functions of several items recited in the claims.

As is described above in greater detail, a computer program may be stored/distributed on a suitable medium, such as an optical storage medium or a solid-state medium supplied together with or as part of other hardware, but may also be distributed in other forms, such as via the Internet or other wired or wireless telecommunication systems.

It should also be understood that, unless clearly indicated to the contrary, in any methods discussed herein that include more than one step or act, the order of the steps or acts of the method is not necessarily limited to the order in which the steps or acts of the method are recited. Further, such methods may include additional or alternative steps or acts. As used in the claims, the mere fact that certain measures are recited in mutually different dependent claims does not indicate that a combination of these measured cannot be used to advantage.

Those skilled in the art will appreciate from the foregoing description that the broad techniques of the embodiments of the present disclosure can be implemented in a variety of forms. Therefore, while the embodiments of this disclosure have been described in connection with particular examples thereof, the true scope of the embodiments of the disclosure should not be so limited since other modifications will become apparent to the skilled practitioner upon a study of the drawings, specification, and following claims.

## Claims

1. A system to detect and correct hemodynamic instability occurring before, during or after a course of hemodynamic measurements in a subject, the system comprising:

   a memory;
   a processing system communicatively coupled to the memory, wherein the processing system is configured to:

   receive first measurement data corresponding to one or more hemodynamic parameters from a hemodynamic measurement system;
   derive one or more hemodynamic parameters from second measurement data;
   determine a hemodynamic instability parameter based on at least one of: i) computing a difference between the first measurement data and the second measurement data and ii) characteristics derived from the second measurement data;
   determine if the hemodynamic instability parameter exceeds a predefined limit; and
   upon determining that the hemodynamic instability parameter exceeds the predefined limit, correct the first measurement data in the hemodynamic measurement system.

2. The system of claim 1, wherein the hemodynamic measurement system is a non-invasive blood pressure measurement (NIBP) measurement system, wherein the NIBP measurement system comprises an NIBP algorithm.

3. The system of claim 2, wherein the NIBP measurement system uses at least one of a Tissue Pressure (TP) signal and patient demographics for measurements.

4. The system of claim 1, wherein the one or more hemodynamic parameters comprise at least one of stroke volume (SV), cardiac output (CO), heart rate (HR), respiration rate (RR), respiration depth, tidal volume (TV) and blood pressure (BP).

5. The system of claim 1, wherein the second measurement data comprises at least one of electrocardiography (ECG) data, bio-impedance data, pulse oximetry data, photoplethysmography (PPG) data, ventilator data, electroencephalography (EEG) data, Tissue Pressure (TP) waveforms, and PPG waveforms.

6. The system of claim 1, wherein the characteristics comprise variations in the one or more hemodynamic parameters derived from the second measurement data.

7. The system of claim 1, wherein the processing system is configured to derive at least one of heart rate from ECG data, pulse rate from TP or PPG waveforms, breathing rate or respiration rate from ECG, bio-impedance, PPG or ventilator data, a measure of tidal volume (TV) or depth of breathing from ECG, bio-impedance, PPG or ventilator data, a change in blood pressure from a change in the pulse arrival time (PAT) as can be measured between the R-peaks in the ECG signal and the cardiac pulses in PPG, bio-impedance or TP signals, and changes in the bispectral index (BIS), which is based on the power distribution of the Fourier transform of the EEG signal.

8. The system of claim 1, wherein the system is configured to report the hemodynamic instability to a user.

9. The system of claim 1, wherein the system is configured to abort and restart a measurement procedure based on the determined hemodynamic instability.

10. A computer-implemented method to detect and correct hemodynamic instability occurring before, during or after a course of hemodynamic measurements in a subject, the method comprising:

receiving first measurement data corresponding to one or more hemodynamic parameters from a hemodynamic measurement system;
deriving one or more hemodynamic parameters from second measurement data;
determining a hemodynamic instability parameter based on at least one of: i) computing a difference between the first measurement data and the second measurement data and ii) characteristics derived from the second measurement data;
determining if the hemodynamic instability parameter exceeds a predefined limit; and
upon determining that the hemodynamic instability parameter exceeds the predefined limit, correcting the first measurement data in the hemodynamic measurement system.

11. The computer-implemented method of claim 10, wherein the one or more hemodynamic parameters comprise at least one of stroke volume (SV), cardiac output (CO), heart rate (HR), respiration rate (RR), respiration depth, tidal volume (TV) and blood pressure (BP).

12. The computer-implemented method of claim 10, wherein the second measurement data comprises at least one of electrocardiography (ECG) data, bio-impedance data, pulse oximetry data, photoplethysmography (PPG) data, ventilator data, electroencephalography (EEG) data, Tissue Pressure (TP) waveforms, and PPG waveforms.

13. The computer-implemented method of claim 10, wherein the characteristics comprise variations in the one or more hemodynamic parameters derived from the second measurement data.

14. The computer-implemented method of claim 10, wherein deriving the one or more hemodynamic parameters from the second measurement data comprises deriving at least one of heart rate from ECG data, pulse rate from TP or PPG waveforms, breathing rate or respiration rate from ECG, bio-impedance, PPG or ventilator data, a measure of tidal volume (TV) or depth of breathing from ECG, bio-impedance, PPG or ventilator data, a change in blood pressure from a change in the pulse arrival time (PAT) as can be measured between the R-peaks in the ECG signal and the cardiac pulses in PPG, bio-impedance or TP signals, and changes in the bispectral index (BIS), which is based on the power distribution of the Fourier transform of the EEG signal.

15. A computer program product to detect and correct hemodynamic instability occurring before, during or after a course of hemodynamic measurements in a subject, the computer program product comprising at least one non-transitory computer-readable storage medium and computer-executable program code for executing the method of claims 10-14.

Fig. 1

*Fig. 2*

*300*

302

304

306

308

310

*Fig. 3*

400

402

404

Fig. 4

*Fig. 5*

*600*

*604*

*602*

*Fig. 6*

Europäisches
Patentamt

European
Patent Office

Office européen
des brevets

# EUROPEAN SEARCH REPORT

Application Number

EP 22 20 4702

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/155207 A1 (LYNN LAWRENCE A [US] ET AL) 13 July 2006 (2006-07-13)<br>* paragraphs [0046], [0053], [0054], [0068], [0221] – [0225]; figures 7, 8, 17 * | 1-15 | INV.<br>A61B5/02<br>A61B5/021<br>A61B5/024<br>A61B5/349<br>G16H50/20 |
| X | EP 1 742 155 A2 (GEN ELECTRIC [FI]) 10 January 2007 (2007-01-10)<br>* paragraphs [0049] – [0058]; figure 1 * | 1,10,15 | |
| A | Anonymous: "IntelliVue Patient Monitor – MP20/30, MP40/50, MP60/70/80/90 – Release D.0 with Software Revision D.00.xx – Patient Monitoring",<br>INSTRUCTIONS FOR USE – IntelliVue Patient Monitor – MP20/30, MP40/50, MP60/70/80/90 – Release D.0 with Software Revision D.00.xx – Patient Monitoring,<br>1 February 2006 (2006-02-01), pages 1-414, XP055578789,<br>DE<br>Retrieved from the Internet:<br>URL:http://its.uvm.edu/FAHC_Alarms/McClure 5/Philips%20Monitor/User%20Manual%20Philip s%20MP50.pdf<br>[retrieved on 2019-04-08]<br>* the whole document * | 1-15 | |
| X | US 2015/265218 A1 (MARTIN GREGORY J [US] ET AL) 24 September 2015 (2015-09-24)<br>* paragraphs [0027], [0073] – [0075], [0094] – [0099]; figures 1a, 2 * | 1,10,15 | TECHNICAL FIELDS SEARCHED (IPC)<br><br>A61B<br>G16H |

The present search report has been drawn up for all claims

| Place of search | Date of completion of the search | Examiner |
|---|---|---|
| Munich | 16 April 2023 | Gentil, Tamara |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 20 4702

This annex lists the patent family members relating to the patent documents cited in the above-mentioned European search report.
The members are as contained in the European Patent Office EDP file on
The European Patent Office is in no way liable for these particulars which are merely given for the purpose of information.

16-04-2023

| Patent document cited in search report | | Publication date | Patent family member(s) | | Publication date |
|---|---|---|---|---|---|
| US 2006155207 | A1 | 13-07-2006 | NONE | | |
| EP 1742155 | A2 | 10-01-2007 | EP | 1742155 A2 | 10-01-2007 |
| | | | US | 2007010723 A1 | 11-01-2007 |
| US 2015265218 | A1 | 24-09-2015 | AU | 2003263966 A1 | 23-02-2004 |
| | | | CA | 2494548 A1 | 12-02-2004 |
| | | | EP | 1538971 A2 | 15-06-2005 |
| | | | JP | 4359562 B2 | 04-11-2009 |
| | | | JP | 2005534423 A | 17-11-2005 |
| | | | KR | 20050044899 A | 13-05-2005 |
| | | | US | 2008064968 A1 | 13-03-2008 |
| | | | US | 2011263991 A1 | 27-10-2011 |
| | | | US | 2015265218 A1 | 24-09-2015 |
| | | | WO | 2004012580 A2 | 12-02-2004 |

EPO FORM P0459

For more details about this annex : see Official Journal of the European Patent Office, No. 12/82

**EP 4 360 543 A1**

**REFERENCES CITED IN THE DESCRIPTION**

**Patent documents cited in the description**

- EP 2953528 B1